# EUROPEAN PATENT APPLICATION

(11) **EP 1 352 608 A2**
(43) Date of publication of application: **15.10.2003**
(21) Application number: 02025653.3
(22) Date of filing: 19.11.2002
(51) Int. Cl.: A61B 5/00, A61B 6/00

(54) **Diagnosis communication method**

(30) Priority: 19.03.2002 JP 2002075355
(71) Applicant: Colin Corporation, Komaki-shi, Aichi-ken (JP)
(72) Inventor: Oka, Tohru, Colin Corporation, Komaki-shi, Aichi-ken (JP); Narimatsu, Kiyoyuki, Colin Corporation, Komaki-shi, Aichi-ken (JP)
(74) Representative: Winter, Brandl, Fürniss, Hübner, Röss, Kaiser, Polte Partnerschaft

(57) **Abstract**

A diagnosis delivering method comprising a physical-information storing step of storing, in a physical-information storing server, a set of physical information obtained from a patient by a physical-information obtaining institution; a physical-information sending step of sending the set of physical information stored in the physical-information storing server at the physical-information storing step, to a doctor's terminal device operable by a doctor so that the doctor makes a diagnosis on the patient based on the physical information; and a diagnosis delivering step of delivering the diagnosis made by the doctor based on the physical information sent at the physical-information sending step, to a patient's terminal device operable by the patient and connected to the doctor's terminal device via a communication line.

## Description

### BACKGROUND OF THE INVENTION

### Field of the Invention

The present invention relates to a diagnosis delivering method of delivering, to a patient, a diagnosis made by a doctor based on the physical information obtained from the patient.

### Related Art Statement

Many patients have their home doctors. However, many home doctors cannot measure or obtain, from their patients, all sorts of physical information needed to make a diagnosis on each patient. For example, usually, a practicing doctor or a small hospital does not have expensive physical-information obtaining devices such as X-ray CT (computerized tomograph) or NMR (nuclear magnetic resonance)-CT. Hence, when a home doctor needs, for making a diagnosis on a patient, one or more sorts of physical information that cannot be obtained by the doctor's own medical devices, the doctor instructs the patient to go to a large medical institution that can obtain the information. The patient goes to the medical institution, and asks the institution to obtain the necessary physical information and make a diagnosis based on it.

However, even if it is needed for a patient to go to a large medical institution so as to obtain the physical information that cannot be obtained by the patient' home doctor who does not have expensive physical-information obtaining devices that can obtain the information, the patient may want the most reliable, home doctor to make a diagnosis based on the information. In this case, the patient may ask the medical institution to only obtain the necessary physical information, and bring the information to the home doctor to hear a diagnosis made by the home doctor based on the information. However, the patient must go twice to the home doctor, and additionally must go to the medical institution. This is a great burden on the patient. On the other hand, if a medical institution to which a patient's home doctor belongs has the above-mentioned expensive physical-information obtaining devices, the patient needs to go only once to the home doctor to hear a diagnosis. This reduces the burden on the patient, on one hand, but increases the burden on the medical institution to which the patient's home doctor belongs, on the other hand.

### SUMMARY OF THE INVENTION

It is therefore an object of the present invention to provide a diagnosis delivering method of making, without having to possess an expensive physical-information obtaining device, a diagnosis based on physical information obtained from a patient by the physical-information obtaining device, and delivering the diagnosis to the patient without additionally costing the patient.

The above object has been achieved by the present invention. According to a first aspect of the present invention, there is provided a diagnosis delivering method comprising a physical-information storing step of storing, in a physical-information storing server, a set of physical information obtained from a patient by a physical-information obtaining institution; a physical-information sending step of sending the set of physical information stored in the physical-information storing server at the physical-information storing step, to a doctor's terminal device operable by a doctor so that the doctor makes a diagnosis on the patient based on the physical information; and a diagnosis delivering step of delivering the diagnosis made by the doctor based on the physical information sent at the physical-information sending step, to a patient's terminal device operable by the patient and connected to the doctor's terminal device via a communication line.

According to this invention, at the physical-information sending step, the physical information obtained by the physical-information obtaining institution and stored in the physical-information storing server is sent to the terminal device of the doctor who needs to make a diagnosis on the patient. Thus, the doctor can make the diagnosis on the patient based on the physical information obtained by an expensive physical-information obtaining that is not possessed by the doctor. In addition, at the diagnosis delivering step, the patient's terminal device and the doctor's terminal device are connected to each other, the diagnosis made based on the physical information is delivered or sent to the patient's terminal device. Thus, the patient can know the diagnosis without having to go to the doctor.

According to a second aspect of the present invention, there is provided a diagnosis delivering method comprising a physical-information storing step of storing, in a physical- information storing server, a set of physical information obtained from a patient by a physical-information obtaining institution; a physical-information sending step of sending the physical information stored in the physical-information storing server at the physical-information storing step, to a doctor's terminal device operable by a doctor so that the doctor makes a diagnosis on the patient based on the physical information; a diagnosis storing step of storing the diagnosis made by the doctor based on the physical information sent at the physical-information sending step, in a diagnosis storing server to which a patient's terminal device operable by the patient is connectable; and a diagnosis delivering step of delivering, according to a diagnosis-request signal sent from the patient's terminal device, the diagnosis stored in the diagnosis storing server at the diagnosis storing step, to the patient's terminal device.

According to this invention, at the physical-information sending step, the physical information obtained by the physical-information obtaining institution and stored in the physical-information storing server is sent to the terminal device of the doctor who needs to make a diagnosis on the patient. Thus, the doctor can make the diagnosis on the patient based on the physical information obtained by an expensive physical-information obtaining that is not possessed by the doctor. In addition, at the diagnosis storing step, the diagnosis made based on the physical information is stored in the diagnosis storing server and, at the diagnosis delivering step, the diagnosis stored in the diagnosis storing server is delivered or sent, according to the diagnosis-request signal sent from the patient's terminal device, to the patient's terminal device. Thus, the patient need not go to the doctor to hear the diagnosis. Moreover, when the diagnosis is delivered to the patient, the doctor need not be present in front of the doctor's terminal device.

According to a preferred feature of the present invention, the physical-information storing step comprises adding the current set of physical information obtained from the patient by the physical-information obtaining institution, to at least one past set of physical information obtained from the patient in past and stored in the physical-information storing server, and the physical-information sending step comprises sending the current set of physical information and the past set of physical information, to the doctor's terminal device.

According to this feature, the doctor can make a diagnosis on the patient while taking, into consideration, not only the current set of physical information of the patient but also the one or more past sets of physical information of the same.

According to another feature of the present invention, the diagnosis delivering method further comprises a standard-physical-information storing step of receiving a set of standard physical information obtained from the patient in a home of the patient, and sent from the patient's terminal device to the physical-information storing server, and adding the received set of standard physical information to the set of physical information stored in the physical-information storing server, and the physical-information sending step comprises sending the set of physical information, and the set of standard physical information added at the standard-physical- information storing step, to the doctor's terminal device.

According to this feature, the doctor can make a diagnosis on the patient while taking, into consideration, not only the set of physical information obtained by the physical-information obtaining institution but also the set of standard physical information obtained in the patient' home.

According to another feature of the present invention, the physical-information sending step comprises sending at least one set of physical information specified by a specifying signal sent from the doctor's terminal device, to the doctor's terminal device.

In the case where the physical-information storing server stores a large amount of past physical information of a patient or many sorts of physical information of a patient, some portion of the information may not be needed for making a diagnosis. If the unnecessary information is sent to the doctor's terminal device, the doctor needs to select the necessary information from all the information sent. Thus, the efficiency of diagnosing is lowered. In contrast thereto, according to this feature, the doctor can select and obtain the necessary information only, and accordingly can make diagnosis with efficiency.

According to a third aspect of the present invention, there is provided a diagnosis storing server for storing a diagnosis made by a doctor based on physical information obtained from a patient by a physical-information obtaining institution, and delivering the diagnosis to a patient's terminal device operable by the patient, according to a diagnosis-request signal sent from the patient's terminal device.

The present diagnosis storing server is preferably employed in the diagnosis delivering method according to the second aspect of the invention.

### BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:
Fig. 1 is a view for explaining the construction of a diagnosis delivering system for carrying out a diagnosis delivering method as a first embodiment of the present invention;
Fig. 2 is a block diagram for explaining various functions of a server shown in Fig. 1;
Fig. 3 is a flow chart representing the functions of the server, explained in Fig. 1;
Fig. 4 is a flow chart representing functions of a patient's terminal device to store and send standard physical information;
Fig. 5 is a flow chart representing a function of a doctor's terminal device, used in the first embodiment, to send a diagnosis;
Fig. 6 is a block diagram for explaining various functions of a server used in a second embodiment of the present invention; and
Fig. 7 is a flow chart representing the functions of the server to store and send a diagnosis.

### DETAILED DESCRIPTION OF PREFERRED EMBODIMENTS

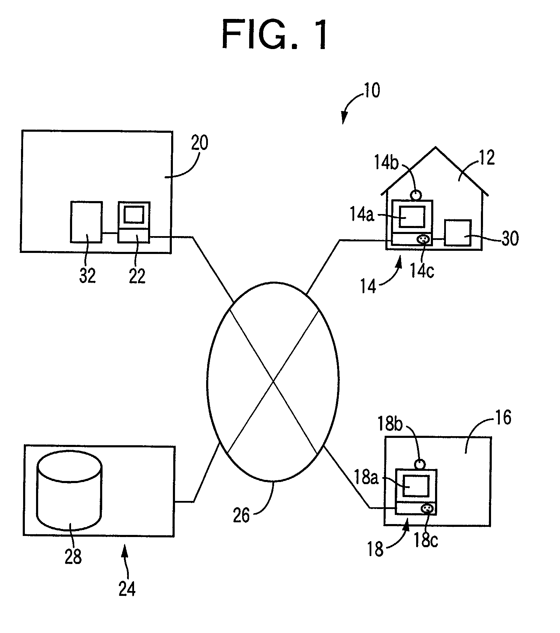

Hereinafter, there will be described a preferred embodiment of the present invention in detail by reference to the drawings. Fig. 1 is a view for explaining the construction of a diagnosis delivering or sending system 10 for carrying out a diagnosis delivering or sending method to which the present invention is applied.

The diagnosis sending system 10 includes a patient's terminal device 14 which is operated by a patient in his or her home 12; a doctor's terminal device 18 which is operated by a doctor belonging to a medical institution 16; a physical-information-obtaining-institution's terminal device 22 which is operated by a person belonging to a physical-information obtaining institution 20 which can obtain various sorts of physical information from a person by using various sorts of physical-information obtaining devices such as X-ray CT or NMR-CT; and a server 24. The patient's terminal device 14, the doctor's terminal device 18, the institution's terminal device 22, and the server 24 are connected to one another via a communication line 26. The server 24 is provided by an electronic computer having a considerably high capacity, and includes a memory device 28 such as a hard disc.

Each of the patient's terminal device 14, the doctor's terminal device 18, and the institution's terminal device 22 may be stationary or portable, and may be provided by a personal computer or a TV set having the function of making communications via an internet. The communication line 26 may be provided a wire or wireless internet or LAN.

The patient's terminal device 14 includes a display portion 14a; an image-taking portion 14b such as a CCD (charge coupled device) camera; a microphone 14c; a memory (not shown) such as a hard disc; and a keyboard (not shown). The images taken by the image-taking portion 14b and the voices and sounds inputted into the microphone 14c are transmitted via the communication line 26, and a diagnosis made by the doctor and received via the line 26 is outputted from the display portion 14a or the microphone 14c. To the patient's terminal device 14, a standard-physical-information obtaining device 30 is connected, for measuring, in the home 12, standard physical information of the patient; such as blood pressure, body weight, body-fat percentage, or body temperature. The standard-physical-information obtaining device 30 includes at least one of a blood pressure measuring device, a body-weight measuring device, a body-fat-percentage measuring device, and a body-temperature measuring device. The standard physical information obtained by the device 30 is stored in the memory of the terminal device 14. The keyboard of the terminal device 14 is operated by the patient to input his or her own standard physical information such as blood pressure, weight, fat percentage, temperature, stature, eye sight, and allergic or non-allergic, and the thus inputted standard physical information obtained by the device 30 is stored in the memory. The patient's terminal device 14 sends, to the server 24, standard physical information, either each time the information is stored in the memory, or periodically at a predetermined period, or when the patient inputs, into the keyboard, a command to send the information.

Also, the doctor's terminal device 18 includes a display portion 18a; an image-taking portion 18b such as a CCD camera; a microphone 18c; a memory (not shown) such as a hard disc; and a keyboard (not shown). The images taken by the image-taking portion 18b and the voices and sounds inputted into the microphone 18c are transmitted via the communication line 26, and the physical information and the images received via the line 26 are outputted from the display portion 18a, and the sounds and voices received via the line 26 are outputted from the microphone 18c.

The physical-information-obtaining-institution's terminal device 22 is connected to a high-priced or advanced physical-information obtaining device 32, such as X-ray CT or NMR-CT, that is disposed in the physical- information-obtaining institution 20, and stores physical information, such as images (i.e., image data), waveforms, or values, obtained (i.e., taken or measured) from each of a plurality of patients by the information obtaining device 32, in the memory thereof, such that the stored physical information is associated with the each patient. In addition, the terminal device 22 sends, to the server 24 via the communication line 26, the physical information together with an identification code identifying the each patient.

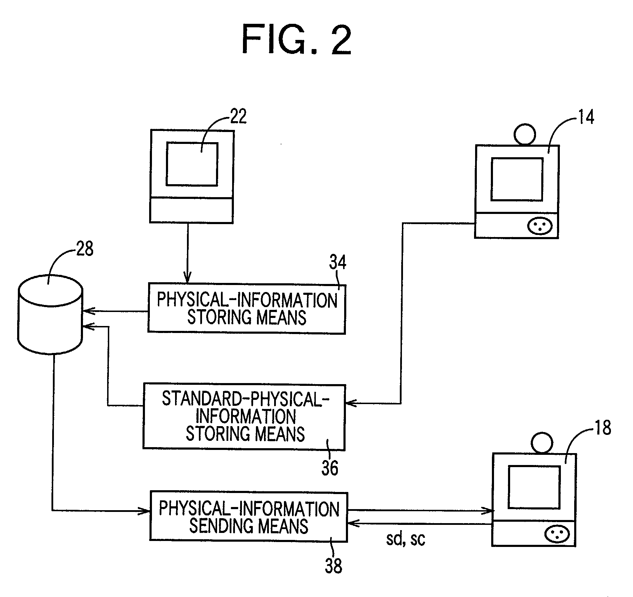

Fig. 2 is a block diagram for explaining various functions of the server 14. A physical-information storing means 34 stores a set of physical information sent from the physical-information-obtaining-institution's terminal device 22, in the memory device 28, such that the set of physical information is associated with each patient based on the identification code sent with the information and identifying the each patient. Thus, the server 24 functions as a physical-information storing server. In the case where the server 24 has stored one or more past sets of physical information of that patient, the server 24 adds the new set of physical information to the past set or sets of physical information.

A standard-physical-information storing means 36 stores a set of standard physical information sent from the patient's terminal device 14 of each patient, in the memory device 28, such that the set of standard physical information is added to the set or sets of physical information of the each patient already stored in the memory device 28.

A physical-information sending means 38 selects, from a lot of physical information stored in the server 24 (or the memory device 28 thereof), the set or sets of physical information of a particular patient specified by a physical-information-request signal, sd, sent from the doctor's terminal device 18 and including identification data identifying the particular patient. The sending means 38 sends the thus selected set or sets of physical information to the doctor's terminal device 18 that has sent the physical-information-request signal sd. In the case where the doctor's terminal device 18 has sent, in addition to the request signal sd, a specifying signal, sc, indicating that the doctor needs physical information of a specified sort only, or physical information obtained in a specified period only, the sending means 38 sends only the physical information specified by the specifying signal sc.

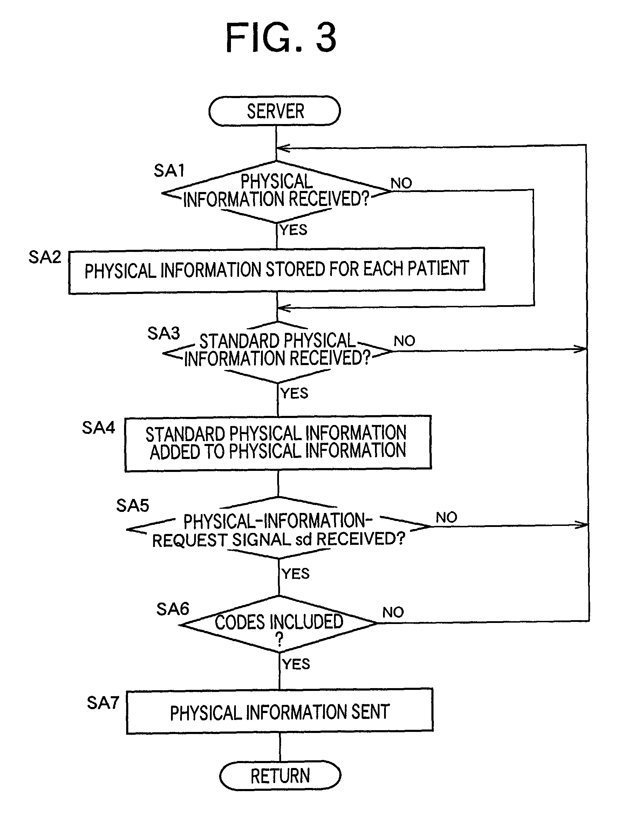

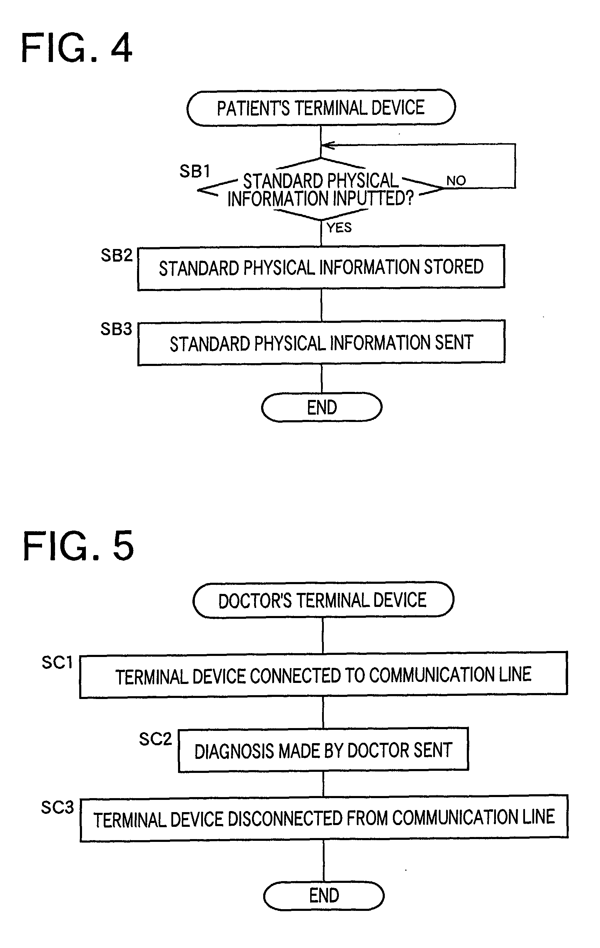

Next, the diagnosis sending method as a first embodiment of the present invention will be described by reference to flow charts shown in Figs. 3, 4, and 5. Fig. 3 shows a flow chart representing the functions of the server 14; Fig. 4 shows a flow chart representing the functions of the patient's terminal device 14 to store and send the standard physical information; and Fig. 5 shows the functions of the doctor's terminal device 18 to send the diagnosis made by the doctor.

First, at Step SA1 of Fig. 3 (hereinafter, "Step(s)" is omitted), the server 14 judges whether the server has received a set of physical information sent from the physical-information-obtaining-institution's terminal device 22. If a negative judgment is made at SA1, the control goes to SA3, described later. On the other hand, a positive judgment is made at SA1, if the patient having the patient's terminal device 14 is instructed by the doctor having the doctor's terminal device 14 to go to the physical-information obtaining institution 20 and request the institution 20 to obtain physical information of a sort specified by the doctor, and the physical-information-obtaining-institution's terminal device 22 sends the thus obtained physical information to the server. Then, the control goes to SA2 corresponding to the physical-information storing means 34 and a physical-information storing step. At SA2, the server stores, in the memory device 28, the thus received physical information such that the physical information is associated with the patient.

At SA3, the server judges whether the server has received a set of standard physical information sent from the patient's terminal device 14. Since the patient's terminal device 14 sends standard physical information according to the flow chart shown in Fig. 4, the flow chart of Fig. 4 will be described first.

At SB1 of Fig. 4, the patient's terminal device 14 judges whether the terminal device 14 has been connected to the standard-physical-information obtaining device 30 and the obtaining device 30 has inputted the standard physical information into the terminal device 14, or whether the standard physical information has been inputted into the terminal device 14 via the keyboard. SB1 is repeated till a positive judgment is made. Meanwhile, if a positive judgment is made at SB1, the control goes to SB2 where the terminal device 14 stores, in the memory device 28, the standard physical information inputted at SB1. Then, the control goes to SB 3 corresponding to a standard-physical-information sending step. At SB3, the terminal device 14 sends, to the server 24, the standard physical information stored at SB2, and the identification code identifying the patient having the terminal device 14.

Back to Fig. 3, if a negative judgment is made at SA3, the control goes back to SA1 and the following steps. Meanwhile, if, at SB3 of Fig. 4, the patient's terminal device 14 sends the standard physical information of the patient to the server, a positive judgment is made at SA3. Then, the control goes to SA4 corresponding to the standard-physical-information storing means 36 and a standard-physical-information storing step. At SA4, the server adds, based on the identification code sent with the standard physical information from the patient's terminal device 14, the standard physical information to the physical information stored in the memory device 28 for the patient identified by the code.

Then, at SA5, the server judges whether the server has received a physical-information-request signal sd sent from the doctor's terminal device 18. If a negative judgment is made at SA5, the control goes back to SA1 and the following steps. On the other hand, if a positive judgment is made at SA5, the control goes to SA6 and SA7 corresponding to the physical-information sending means 38 and a physical-information sending step.

At SA6, the server judges whether the received physical-information-request signal sd includes an identification code identifying the particular doctor having the doctor's terminal device 18, and a permission code indicating that the patient has permitted the particular doctor to obtain the physical information of the patient. If the request signal sd does not include the doctor's identification code or the permission code, or if at least one of those two codes included in the request signal sd is not correct, a negative judgment is made at SA6, and the server does not send the physical information of the patient. Then, the control goes back to SA1 and the following steps. Thus, the secrecy of the physical information of the patient is assured.

On the other hand, if a positive judgment is made at SA6, the control goes to, SA7 where the server sends, to the doctor's terminal device 18 that has sent the physical-information-request signal sd, the physical information of the patient identified by the request signal sd. If the server 24 stores one or more past sets of physical information of the patient identified, then the server sends all the sets of physical information of the patient, including both the last set of physical information and the past set or sets of physical information. By the way, if at SA5 the server has received not only the request signal sd but also the specifying signal sc, the server sends, to the doctor's terminal device 18, only the physical information specified by the specifying signal sc. However, the server can receive the request signal sd before SA1 where the server receives physical information. Therefore, immediately after the doctor instructs the patient to go to the physical-information-obtaining institution 20 and request the institution 20 to obtain his or her physical information, the doctor can send the request signal sd to the server 24. In this case, the doctor can obtain the physical information of the patient immediately after the server 24 has received the information from the institution 22.

At SA7, the server sends the physical information to the doctor's terminal device 18, so that the doctor can make a diagnosis based on the physical information. The doctor sends the diagnosis to the patient via the doctor's terminal device 18 and the patient's terminal device 14. Next, the function of the doctor's terminal device 18 to send diagnosis will be described by reference to Fig. 5. First, at SC1, the doctor's terminal device 18 is connected to the patient's terminal device 14 via the communication line 26. In this state, the doctor explains the diagnosis in front of the doctor's terminal device 18. The patient is required to be present in front of the patient's terminal device 18. When the doctor explains the diagnosis in front of the doctor's terminal device 18, the images of the doctor who is explaining the diagnosis, are taken by the image-taking portion 18b, and the voices and sounds produced by the doctor are inputted into the microphone 18c, and the thus taken images and the thus inputted voices and sounds, i.e., the diagnosis, are outputted from the display portion 14a and the microphone 14c of the patient's terminal device 14, respectively. This step corresponds to the diagnosis sending step at SC2.

After the doctor has sent the diagnosis, he or she operates the doctor's terminal device 18 to disconnect the terminal device 18 from the patient's terminal device 14.

In the illustrated embodiment, at SA6 and SA7 (the physical-information sending step), the physical information of the patient obtained by the physical-information obtaining institution 20 and stored by the server 24 is sent to the terminal device 18 of the doctor who needs to make a diagnosis on the patient. Thus, the doctor can make the diagnosis based on the physical information obtained by the physical-information obtaining device 32 possessed by the institution 20, and accordingly the doctor need not possess the expensive device 32. In addition, at SC2 (the diagnosis sending step), the doctor's terminal device 18 is connected to the patient's terminal device 14, so that the diagnosis made based on the physical information is sent to the patient's terminal device 18. Thus, the patient can obtain the diagnosis without having to go to the doctor after the institution 20 has obtained the physical information from the patient.

In addition, in the illustrated embodiment, the server 24 stores the past sets of physical information of the patient and, at SA6 and SA7 (the physical-information sending step), the server sends, to the doctor, not only the current set of physical information but also the past sets of physical information. Therefore, the doctor can make the diagnosis while taking into consideration the past sets of physical information in addition to the current set of physical information.

Also, in the illustrated embodiment, at SA6 and SA7 (the physical-information sending step), the server 24 sends, to the doctor's terminal device 18, the physical information of the patient including the standard physical information obtained from him or her in his or her home 12. Therefore, the doctor can make the diagnosis while taking into consideration the standard physical information obtained in the patient's home 12 in addition to the physical information obtained in the physical-information-obtaining institution 20.

Also, in the illustrated embodiment, at SA6 and SA7 (the physical-information sending step), the server 24 sends, to the doctor's terminal device 18, only the physical information specified by the specifying signal sc sent from the terminal device 18. Thus, the doctor can obtain the physical information as needed to make diagnosis and accordingly can make the diagnosis with efficiency.

Next, there will be described a second embodiment of the present invention. The same reference numerals as used in the first embodiment are used to designate the corresponding elements employed in the second embodiment, and the description of those elements are omitted.

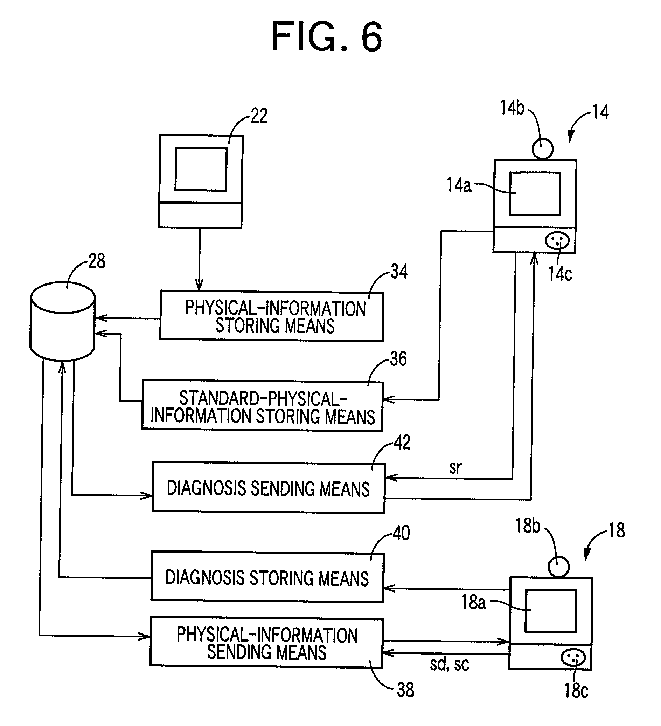

The second embodiment is identical with the first embodiment with respect the steps up to the step where the doctor obtains the physical information of the patient, but is different from the first embodiment with respect to the step where the doctor sends, to the patient, the diagnosis made based on the obtained physical information. The same diagnosis sending system as shown in Fig. 1 is used for carrying out the diagnosis sending method as the second embodiment. However, respective functions of a patient's terminal device 14, a doctor's terminal device 18, and a server 24 employed in the second embodiment differ from those of the counterparts 14, 18, 24 employed in the first embodiment. Fig. 6 is a block diagram for explaining various functions of the server 24 in the second embodiment.

In the second embodiment, a diagnosis that is made by a doctor based on physical information sent to the doctor's terminal device 18 by a physical-information sending means 38, that is, images of the doctor taken by an image-taking device 18b and voices and sounds produced by the doctor and inputted into a microphone 18c are converted into electronic data and sent to the server 24. In the server 24, a diagnosis storing means 40 stores, in a memory device 28, the diagnosis sent from the doctor's terminal device 18. Thus, in the second embodiment, the server 24 functions as not only a physical-information storing server but also a diagnosis storing server.

A diagnosis sending means 42 sends, based on a diagnosis-request signal, sr, sent from the patient's terminal device 14, the diagnosis stored in the memory device 28 by the diagnosis storing means 40, to the patient's terminal device 14.

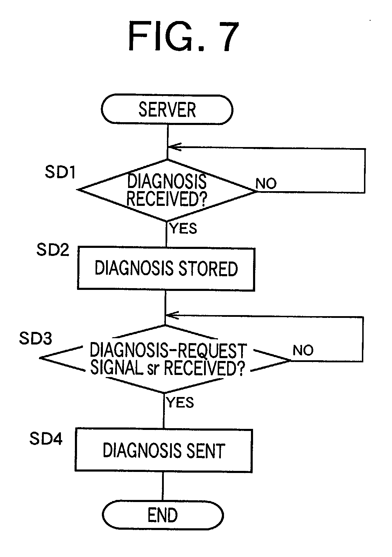

Fig. 7 is a flow chart for explaining the functions of the server 24 to store and send diagnosis. First, at SD1, the server judges whether the server has received a diagnosis sent from the doctor's terminal device 18. SD1 is repeated until a positive judgment is made. Meanwhile, if a positive judgment is made at SD1, the control goes to SD2 corresponding to the diagnosis storing means 40 and a diagnosis storing step. At SD2, the server stores, in the memory device 28, the diagnosis sent from the doctor's terminal device 18.

Then, the control goes to SD3 and SD4 corresponding to the diagnosis sending means 42 and a diagnosis sending step. First, at SD3, the server judges whether the server has received a diagnosis-request signal sr sent from the patient's terminal device 14. SD3 is repeated until a positive judgment is made. The diagnosis-request signal sr includes an identification code identifying a patient. If the patient identified by the identification code is not identical with the patient on whom the doctor has made the diagnosis, a negative judgment is made at SD3.

Meanwhile, if a positive judgment is made at SD3, the control goes to SD4 to send the diagnosis stored in the memory device 28, to the patient's terminal device 14 that has sent the diagnosis-request signal sr. Thus, the diagnosis made by the doctor is outputted from the display portion 14a and the microphone 14c of the terminal device 14.

In the second embodiment, at SA6 and SA7 (the physical-information sending step), the physical information of the patient obtained by the physical-information obtaining institution 20 and stored by the server 24 is sent to the terminal device 18 of the doctor who needs to make a diagnosis on the patient. Thus, the doctor can make the diagnosis based on the physical information obtained by the physical-information obtaining device 32 possessed by the institution 20, and accordingly the doctor need not possess the expensive device 32. In addition, at SD2 (the diagnosis storing step), the diagnosis made based on the physical information is stored in the server 24 and, at SD3 and SD4 (the diagnosis sending step), the server 24 sends, according to the diagnosis-request signal sr sent from the patient's terminal device 14, the diagnosis stored therein to the patient's terminal device 14. Thus, the patient need not go to the doctor to hear the diagnosis from him or her. Moreover, when the diagnosis is sent to the patient, the doctor need not be present in front of the doctor's terminal device 18. That is, the doctor can send, to the server 24, the diagnosis at his or her convenient time, and the patient can receive, from the server 24, the diagnosis at his or her convenient time.

While the present invention has been described in its embodiment by reference to the drawings, it is to be understood that the present invention can otherwise be embodied.

For example, in each of the first and second embodiments, the patient having the patient's terminal device 14 and the doctor having the doctor's terminal device 18 are registered as members or subscribers in the server 24.However, the diagnosis sending system or method may be used by patients and doctors who are not registered in the server 24.

In each of the first and second embodiments, the server 24 functions as the physical-information storing server that stores the physical information sent from the physical-information-obtaining-institution's terminal device 22. However, the institution's terminal device 22 may functions as the physical-information storing server.

In each of the first and second embodiments, the server 24 functions as the diagnosis storing server that stores the diagnosis sent from the doctor's terminal device 18. However, the doctor's terminal device 18 may functions as the diagnosis storing server.

In each of the first and second embodiments, diagnosis is represented by images, and voices and sounds. However, diagnosis may be represented by letters only.

It is to be understood that the present invention may be embodied with various changes or improvements that may occur to a person skilled in the art without departing from the scope and spirit of the present invention.

## Claims

1. A diagnosis delivering method comprising:
a physical-information storing step of storing, in a physical-information storing server, a set of physical information obtained from a patient by a physical-information obtaining institution;
a physical-information sending step of sending the set of physical information stored in the physical-information storing server at the physical-information storing step, to a doctor's terminal device operable by a doctor so that the doctor makes a diagnosis on the patient based on the physical information; and
a diagnosis delivering step of delivering the diagnosis made by the doctor based on the physical information sent at the physical-information sending step, to a patient's terminal device operable by the patient and connected to the doctor's terminal device via a communication line.

2. A diagnosis delivering method comprising:
a physical-information storing step of storing, in a physical-information storing server, a set of physical information obtained from a patient by a physical-information obtaining institution;
a physical-information sending step of sending the physical information stored in the physical-information storing server at the physical-information storing step, to a doctor's terminal device operable by a doctor so that the doctor makes a diagnosis on the patient based on the physical information;
a diagnosis storing step of storing the diagnosis made by the doctor based on the physical information sent at the physical-information sending step, in a diagnosis storing server to which a patient's terminal device operable by the patient is connectable; and
a diagnosis delivering step of delivering, according to a diagnosis-request signal sent from the patient's terminal device, the diagnosis stored in the diagnosis storing server at the diagnosis storing step, to the patient's terminal device.

3. A method according to claim 1 or claim 2, wherein the physical-information storing step comprises adding the current set of physical information obtained from the patient by the physical-information obtaining institution, to at least one past set of physical information obtained from the patient in past and stored in the physical-information storing server, and
wherein the physical-information sending step comprises sending the current set of physical information and the past set of physical information, to the doctor's terminal device.

4. A method according to claim 1 or claim 2, further comprising a standard-physical-information storing step of receiving a set of standard physical information obtained from the patient in a home of the patient, and sent from the patient's terminal device to the physical-information storing server, and adding the received set of standard physical information to the set of physical information stored in the physical-information storing server,
wherein the physical-information sending step comprises sending the set of physical information, and the set of standard physical information added at the standard-physical-information storing step, to the doctor's terminal device.

5. A method according to claim 3 or claim 4, wherein the physical-information sending step comprises sending at least one set of physical information specified by a specifying signal sent from the doctor's terminal device, to the doctor's terminal device.

6. A diagnosis storing server (24, 18) for storing a diagnosis made by a doctor based on physical information obtained from a patient by a physical-information obtaining institution (20, 22), and delivering the diagnosis to a patient's terminal device (14) operable by the patient, according to a diagnosis-request signal sent from the patient's terminal device.
